# EUROPÄISCHE PATENTSCHRIFT

(11) **EP 3 021 889 B1**
(45) Veröffentlichungstag und Bekanntmachung des Hinweises auf die Patenterteilung: **01.07.2020**
(21) Anmeldenummer: 14739432.4
(22) Anmeldetag: 15.07.2014
(51) Int. Cl.: A61M 1/36, A61M 39/28, A61M 1/26, A61M 1/16

(54) **STEUER- ODER REGELVORRICHTUNG, KONFIGURIERT, UM EIN VERFAHREN ZUM STEUERN EINER BLUTBEHANDLUNGSVORRICHTUNG**
A CONTROL DEVICE CONFIGURED TO CONTROL A METHOD FOR CONTROLLING A BLOOD TREATMENT APPARATUS
DISPOSITIF DE PILOTAGE POUR CONTROLER UN PROCÉDÉ DE COMMANDE D'UN DISPOSITIF DE TRAITEMENT DU SANG

(30) Priorität: 15.07.2013 DE 102013011717
(43) Veröffentlichungstag der Anmeldung: 25.05.2016
(73) Patentinhaber: Fresenius Medical Care Deutschland GmbH, 61352 Bad Homburg (DE)
(72) Erfinder: PARISOTTO, Maria Teresa, 61350 Bad Homburg (DE); WEHMEYER, Wolfgang, 72076 Tübingen (DE)
(74) Vertreter: Bobbert & Partner Patentanwälte PartmbB
(86) Internationale Anmeldenummer: PCT/EP2014/065121
(87) Internationale Veröffentlichungsnummer: WO 2015/007722

(56) Entgegenhaltungen:
- EP-A1- 2 564 884
- EP-A1- 2 564 884
- WO-A1-2011/063923
- WO-A1-2011/063923
- WO-A1-2013/024825
- WO-A1-2013/024825
- DE-A1- 3 442 744
- DE-T2- 60 224 387

## Beschreibung

Die vorliegende Erfindung eine Steuer- oder Regelvorrichtung, konfiguriert, um ein Verfahren zum Steuern einer Blutbehandlungsvorrichtung, insbesondere nach Abschluss der Blutbehandlung eines Patienten, gemäß Anspruch 1. Sie betrifft ferner eine Steuer- oder Regelvorrichtung gemäß Anspruch 8, eine Blutbehandlungsvorrichtung gemäß Anspruch 9, ein Computerprogramm gemäß Anspruch 11, ein Computerprogramm-Produkt gemäß Anspruch 12 und ein digitales Speichermedium gemäß Anspruch 13.

Aus der Praxis sind Verfahren zum Steuern einer Blutbehandlungsvorrichtung bekannt, u. a. um diese sowie weitere Einrichtungen nach Abschluss der Behandlung eines Patienten, welche mit der Blutbehandlungsvorrichtung und den weiteren Einrichtungen erfolgt ist, durch Spülen, Reinigen usw. in einen bestimmten Zustand zu versetzen.

Eine Aufgabe der vorliegenden Erfindung ist es, ein weiteres Verfahren zum Steuern einer Blutbehandlungsvorrichtung vorzuschlagen. Ferner sollen eine Steuer- oder Regelvorrichtung und eine Blutbehandlungsvorrichtung angegeben werden. Zudem soll ein Computerprogramm, ein Computerprogramm-Produkt und ein digitales Speichermedium vorgeschlagen werden.

Die erfindungsgemäße Aufgabe kann durch ein Verfahren mit den Merkmalen des Anspruchs 1 gelöst werden. Sie kann ferner durch eine Steuer- oder Regelvorrichtung mit den Merkmalen des Anspruchs 8, einer Blutbehandlungsvorrichtung mit den Merkmalen des Anspruchs 9, einem Computerprogramm mit den Merkmalen des Anspruchs 11, ein Computerprogramm-Produkt mit den Merkmalen des Anspruchs 12 und ein digitales Speichermedium mit den Merkmalen des Anspruchs 13 gelöst werden.

Erfindungsgemäß wird somit ein automatisches Verfahren vorgeschlagen zum Steuern einer Blutbehandlungsvorrichtung, insbesondere nach Abschluss einer mittels der Blutbehandlungsvorrichtung durchgeführten Blutbehandlung. Die Blutbehandlungsvorrichtung weist eine Blutpumpe zum Fördern von sich innerhalb eines extrakorporalen Blutkreislaufs befindenden Bluts auf. Der Blutschlauchsatz weist einen arteriellen Abschnitt auf, welcher eine arterielle Nadel aufweist oder hiermit verbunden ist, und welcher eine arterielle Patientenschlauchklemme aufweist. Der Blutschlauchsatz weist ferner einen venösen Abschnitt auf, welcher eine venöse Nadel aufweist oder hiermit verbunden ist, und welcher eine venöse Patientenschlauchklemme aufweist. Zwischen dem arteriellen und dem venösen Abschnitt ist wenigstens ein Blutfilter angeordnet. Dieser weist eine Membran auf. Die Membran trennt eine Blutkammer von einer Dialysierflüssigkeitskammer.

Das erfindungsgemäße Verfahren umfasst manche oder alle der folgenden Schritte in beliebiger Kombination:
a) Schließen der arteriellen Patientenschlauchklemme und der venösen Patientenschlauchklemme;
b) Fördern von Dialysierflüssigkeit mittels der Dialysierflüssigkeitspumpe;
c) Öffnen der venösen Patientenschlauchklemme;
d) Schließen der Dialysatablaufleitung;
e) Unterbinden des Dialysierflüssigkeitsflusses, insbesondere sobald ein vorgegebenes erstes Volumen abzüglich eines zweiten Volumens über die Membran übergetreten oder mittels Dialysierflüssigkeitspumpe gefördert ist, wobei das erste Volumen beispielsweise dem gesamten Volumen entspricht, den der extrakorporale Blutkreislauf auf Blutseite umfasst (etwa sein Fassungsvermögen oder sein Primingvolumen), und wobei das zweite Volumen beispielsweise ebenfalls vorbestimmt ist oder beispielsweise dem Volumen des arteriellen Abschnitts des extrakorporalen Blutkreislaufs bis zum Eingang des Blutfilters oder bis einschließlich des Fassungsvermögens der Blutkammer des Blutfilters entsprechen kann; das vorgegebene erste Volumen abzüglich des zweiten Volumens entspricht in bestimmten erfindungsgemäßen Ausführungsformen dem Primingvolumen oder Fassungsvermögen des arteriellen Abschnitts des verwendeten extrakorporalen Blutkreislaufs (wahlweise mit oder ohne Fassungsvermögen der Blutkammer des Blutfilters);
f) Schließen der venösen Patientenschlauchklemme;
g) Fördern von Dialysierflüssigkeit mittels der Dialysierflüssigkeitspumpe;
h) Öffnen der arteriellen Patientenschlauchklemme bei weiterhin geschlossener Dialysatablaufleitung;
i) optional: Verringern der Fördergeschwindigkeit durch die Dialysierflüssigkeitspumpe;
j) Schließen der arteriellen Patientenschlauchklemme;
k) Stoppen der Dialysierflüssigkeitspumpe sobald das zweite Volumen gefördert wurde oder Luft oder Gerinnsel mittels eines Sensors, insbesondere mittels des Luftblasendetektors stromaufwärts der arteriellen Patientenschlauchklemme, erkannt wurde, oder sobald ein anderes, vorbestimmte Kriterium erfüllt ist (etwa das Ergebnis einer Sensormessung); Das zweite Volumen kann dem arteriellen *rinse back*-Volumen entsprechen.

Das erfindungsgemäße Verfahren umfasst weiter optional die Schritte l) bis q), oder alternativ die Schritte r) bis s), oder beliebige Unterkombinationen hiervon in beliebiger Kombination:
l) Anfahren einer stromab des Blutfilters liegenden Dialysierflüssigkeitspumpe, oder einer stromauf des Blutfilters liegenden Dialysierflüssigkeitspumpe, insbesondere rückwärts;
m) Schließen, Geschlossenhalten oder Öffnen der Dialysierflüssigkeitszulaufsleitung;
n) Öffnen, Offenhalten oder Schließen der Dialysatsablaufleitungsklemme;
o) Schließen oder Geschlossenhalten der arteriellen und der venösen Patientenschlauchklemme;
p) Öffnen eines mit der venösen Blutkammer in Verbindung stehenden oder daran vorgesehenen Belüftungsventils, beispielsweise in einer Verbindungsleitung mit einem venösen Drucksensor oder zwischen einem venösen Druckwandler und einem venösen Drucksensor;
q) Saugen von Dialysierflüssigkeit mittels der Dialysierflüssigkeitspumpe über die Membran aus dem Blutschlauchsatz;
r) Öffnen der Dialysatablaufleitung, so dass ein Durchströmen seines Lumens oder ein Verwerfen von Flüssigkeit über die Dialysatablaufleitung, beispielsweise via *drain,* möglich ist;
s) Einbringen von Gas, beispielsweise Luft, in die Verbindungsleitung, beispielsweise zwischen einen venösen Druckwandler und einen venösen Drucksensor. Das Gas kann beispielsweise mittels Kompressor eingebracht werden. Das Gas verdrängt das Dialysat über die Membran auf die Dialysatausgangsseite, wo es in den Abfluss geführt wird;

Das erfindungsgemäße Verfahren umfasst weiter optional wenigstens einen oder mehrere der folgenden Schritte:
t) optional: Entleeren des Behälters mit Bikarbonat-Trockenkonzentrat, beispielsweise via *drain,* beispielsweise zum Verwerfen des verbliebenen Beutelinhalts;
u) Stoppen der Dialysierflüssigkeitspumpe;
v) Schließen von arterieller Patientenschlauchklemme, venöser Patientenschlauchklemme, ggf. ferner auch der Substituatklemme (oder Infusatklemme) zum Verschließen einer Leitung zum Zuführen von Substituat, soweit noch nicht erfolgt; und
w) Optional: Ausgeben eines, insbesondere seit Beginn des erfindungsgemäßen Verfahrens erstmaligen, Alarms oder Hinweises zum Informieren des Dialysepersonals über das Verfahrensende.

Die Schritte t) bis w) können vor, parallel zu oder nach den Schritten l) bis s) (soweit vorgesehen) oder deren Beginn starten oder vollends ablaufen.

Die Reihenfolge insbesondere der Schritte t) bis w) kann wie hier angeführt sein. Diese Schritte können, sofern jeweils vorgesehen, aber auch in anderer, beliebiger Reihenfolge ablaufen. Dasselbe gilt in einigen erfindungsgemäßen Ausführungsformen für alle erfindungsgemäßen Schritte. Auch sie können, wo für den Fachmann erkennbar technisch möglich, in beliebigen anderen Abfolgen als den hierin beschriebenen ablaufen und fallen auch dann noch unter die vorliegende Erfindung.

Zwischen die Schritte f) und g) kann sich ein weiterer Schritt einfügen, welcher zum Erzeugen oder Sicherstellen eines durchgängigen Pumpschlauchsegments dient. Diese Durchgängigkeit kann beispielsweise, wie weiter unten ausgeführt, erzielt werden, indem das Pumpschlauchsegment außer Eingriff mit der Pumpe gebracht wird, welche meist eine okkludierende Pumpe ist. Hierzu kann das Pumpschlauchsegment, also der mit den bewegten Pumpkörpers der Pumpe in Kontakt stehende Schlauchabschnitt, ausgeworfen werden. Letzteres kann beispielsweise durch Öffnen, Lösen oder Auswerfen des weiter unten erläuterten sog. ALPHA-Clips erfolgen. Dieser Verfahrensschritt ist weiter unten als Schritt f1) bezeichnet. Ein durchgängiges Pumpschlauchsegment kann ferner erzielt oder sichergestellt werden, indem in einem hier als f2) bezeichneten Verfahrensschritt die mit dem Pumpschlauchsegment in Eingriff stehende Pumpe entgegen ihrer üblichen Förderrichtung, d.h. rückwärts, gedreht wird, insbesondere synchron zur Dialysierflüssigkeitspumpe.

Das optionale Verringern der Fördergeschwindigkeit durch die Dialysierflüssigkeitspumpe gemäß Verfahrensschritt i) kann ein Verringern der Fördergeschwindigkeit auf 20%, 30%, 40%, 50%, 60%, 70% oder 80% der üblichen oder durchschnittlichen Fördergeschwindigkeit während der Behandlung bedeuten, vorzugsweise deren Halbierung.

Der Begriff "stromab" oder "stromabwärts" bezieht sich hierin auf die (Blut- oder Dialysierflüssigkeits-)Strömungsrichtung während der Behandlung des Patienten. Der arterielle Bereich des Blutschlauchsatzes liegt somit stromaufwärts des Blutfilters. Der venöse Bereich des Blutschlauchsatzes liegt folglich stromabwärts des Blutfilters.

Die erfindungsgemäße Steuer- oder Regelvorrichtung ist eingerichtet, konfiguriert, bestimmt und/oder programmiert, um im Zusammenspiel mit einer mit den hierzu erforderlichen Einrichtungen ausgestatteten Blutbehandlungsvorrichtung das erfindungsgemäße Verfahren auszuführen oder sein Ausführen zu veranlassen.

Die erfindungsgemäße Blutbehandlungsvorrichtung weist eine erfindungsgemäße Steuer- oder Regelvorrichtung auf oder ist hiermit verbunden.

Das erfindungsgemäße Computerprogramm kann direkt in den internen Speicher eines digitalen Computers geladen werden und umfasst Softwarecodeabschnitte, mit denen die Schritte des erfindungsgemäßen Verfahrens ausgeführt werden oder ausführbar sind, wenn das Computerprogramm auf einem Computer läuft.

Das erfindungsgemäße digitales Speichermedium weist elektrisch auslesbare Steuersignale auf, die derart mit einem programmierbaren Computersystem oder Computer zusammenwirken können, dass die Schritte des erfindungsgemäßen Verfahrens veranlasst werden.

Ein erfindungsgemäßes, insbesondere digitales, insbesondere nicht-flüchtiges, Speichermedium (hier auch als Träger bezeichnet), insbesondere in Form von Diskette, RAM, ROM, CD, Festplatte, DVD, USB-Stick, Flashcard, SD-Karte oder EPROM, insbesondere mit elektronisch oder optisch auslesbaren Steuersignalen, kann derart mit einem programmierbaren Computer oder Computersystem zusammenwirken, dass die maschinellen Schritte eines hierin beschriebenen erfindungsgemäßen Verfahrens veranlasst werden.

Dabei können alle, einige oder manche der maschinell durchgeführten Schritte des erfindungsgemäßen Verfahrens veranlasst werden.

Ein erfindungsgemäßes Computerprogramm-Produkt weist einen auf einem maschinenlesbaren Träger oder Speichermedium gespeicherten Programmcode auf zur Veranlassung der Schritte des erfindungsgemäßen Verfahrens, wenn das Computerprogramm-Produkt auf einem Rechner abläuft. Unter einem Computerprogramm-Produkt kann erfindungsgemäß beispielsweise ein auf einem Träger gespeichertes Computerprogramm, ein Embedded System als umfassendes System mit einem Computerprogramm (z. B. ein elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Ein maschinenlesbarer Träger bezeichnet in bestimmten Ausführungsformen der vorliegenden Erfindung einen Träger, der mittels Software und/oder Hardware interpretierbare Daten oder Informationen enthält. Der Träger kann eine Diskette, eine CD, DVD, ein USB-Stick, eine Flashcard, eine SD-Karte und dergleichen sein.

Ein erfindungsgemäßes Computerprogramm weist einen Programmcode auf zur Veranlassung der maschinellen Schritte eines erfindungsgemäßen Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Auch für das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden.

Bei allen vorstehenden und folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

Bei der venösen Patientenschlauchklemme handelt es sich in manchen erfindungsgemäßen Ausführungsformen um jede Einrichtung zum Unterbrechen des Flusses im venösen Abschnitt des Blutschlauchsatzes stromab des Blutfilter.

Bei der arteriellen Patientenschlauchklemme handelt es sich in einigen erfindungsgemäßen Ausführungsformen um jede Art von Einrichtungen zum Unterbrechen des Flusses im arteriellen Abschnitt des Blutschlauchsatzes stromab des Blutfilter.

In bestimmten erfindungsgemäßen Ausführungsformen umfasst das Verfahren das Stoppen der Blutpumpe zum Beenden einer dem vorliegenden Verfahren vorangegangenen Blutbehandlung, wenn eine der folgenden Bedingungen erfüllt ist:
- Erreichen eines vorgegebenen Zeitpunkts (z.B. einer Uhrzeit);
- Erreichen einer vorgegebenen Behandlungsdauer, beispielsweise der effektiven Dialysezeit (also die Zeitdauer, während welcher der Dialysator oder Blutfilter von Dialysierflüssigkeit durchströmt wurde, oder während welcher dem Blut Filtrat abgezogen wurde; Zeiten isoliert erfolgender Ultrafiltration zählen in manchen erfindungsgemäßen Ausführungsformen nicht zur effektiven Dialysezeit);
- Erreichen eines vorgegebenen Dialysedosiswerts (beispielsweise dem Verhältnis Kt/V (nach Gotch und Sargent, beispielsweise mit K als Maß für die Clearance, t als Dauer der effektiven Dialysezeit in Minuten, und V als Angabe von 60 % der Körpermasse (Gewicht), in der das Blut zirkulieren kann (Körperwassergehalt); oder
- Erreichen einer vorbestimmten Ultrafiltrationsmenge.

In manchen dieser erfindungsgemäßen Ausführungsformen ergeht kein Alarm oder keine Mitteilung aufgrund des Stoppens der Blutpumpe, da die Mitwirkung des Personals bis auf weiteres nicht erforderlich ist. In bestimmten dieser erfindungsgemäßen Ausführungsformen umfasst das Verfahren das Überwachen, ob eines der vorstehend genannten Kriterien erfüllt ist. Entsprechende Sensoren, Zeitmesser oder andere Einrichtungen sind vorhanden.

In bestimmten erfindungsgemäßen Ausführungsformen ergeht kein Alarm oder keine Mitteilung an das Personal zwischen jeweils allen oder manchen der aufeinanderfolgenden Schritte. Die Blutbehandlungsvorrichtung vollzieht in diesen erfindungsgemäßen Ausführungsformen die betreffenden Schritte und deren Übergänge in der vorgegebenen Reihenfolge von sich aus oder automatisch. Es bedarf in diesen erfindungsgemäßen Ausführungsformen keiner Mitwirkung des Personals. Wie auch immer ausgestaltete Hinweise (optisch, akustisch, usw. sowie Kombinationen hiervon) an das Personal über den Verfahrensstatus sind in diesen erfindungsgemäßen Ausführungsformen daher entbehrlich und können entfallen, obgleich derartige Hinweise optional vorgesehen sein können.

In manchen erfindungsgemäßen Ausführungsformen umfasst das Verfahren ein automatisches Auswerfen des Blutschlauchsegments des Blutschlauchsatzes durch eine Einrichtung, beispielsweise mittels des sog. ALPHA-Clips, wie er beispielsweise in WO 2005/111424 A1 der Anmelderin der vorliegenden Erfindung als Adapter 32 bzw. Anschlussstück 14 offenbart ist, deren diesbezüglicher Inhalt hiermit mittels Verweis vollumfänglich aufgenommen wird.

In einigen erfindungsgemäßen Ausführungsformen des Verfahrens wird in Schritt b) die Dialyierflüssigkeitsrate automatisch verändert. Die Veränderung kann ein Absenken einer zuvor eingestellten Dialysierflüssigkeitsrate sein, sie kann eine an- und abschwellende oder anderweitige Veränderung sein, beispielsweise eine, die zu einem Pulsieren des Dialysierflüssigkeitsstroms führt. Dies kann vorteilhaft ein Lösen von Zell- oder Proteinbestandteilen im Filter begünstigen oder bewirken.

In manchen erfindungsgemäßen Ausführungsformen umfasst das Verfahren einen oder mehrere der folgenden, jeweils automatisch oder manuell durchgeführten Schritte:
- Trennen der Dialysatkonnektoren (Verbindungsabschnitte, mit welchen der Blutfilter auf Seiten seiner Dialysierflüssigkeitskammer mit der Hydraulik der Blutbehandlungsvorrichtung lösbar verbunden wird) und/oder eines online Konnektors, oder Unterbinden einer Fluidkommunikation derselben mit der Hydraulik mittels Ventilen; auf diese Weise erfolgt ein Umschalten des Blutschlauchsatzes in eine bypass-Stellung, d. h. die Dialysatkonnektoren (jetzt flüssigkeitsfrei, da leer gesaugt) sowie der online Konnektor werden von der Hydraulik durch Ventile getrennt.
- Trennen des Behälters mit Bikarbonat-Trockenkonzentrat von der Hydraulik oder Unterbinden einer Fluidkommunikation zwischen Behälter und Hydraulik;
- Das Desinfizieren erfolgt bis maschinenseitig erkannt wird, dass die Spülung der nicht verbundenen Hydraulikteile notwendig ist und die Behandlungsvorrichtung in den sog. *HOLD status* übergeht.

In manchen erfindungsgemäßen Ausführungsformen umfasst das Verfahren eine automatisch ausgelöste Blutdruckmessung. Die Ergebnisse können weitergeleitet werden. Eine Auswertung derselben ist nicht Bestandteil des vorliegenden Verfahrens. In manchen erfindungsgemäßen Ausführungsformen umfasst das Verfahren ein Überprüfen, ob die Dialysatablaufleitung und die Dialysierflüssigkeitszulaufleitung an der Maschine konnektiert wurden, ob der online-Konnektor in Spülstellung ist und/oder ob der Behälter mit Bikarbonat-Trockenkonzentrat entfernt wurde. Sind eine oder alle dieser Überprüfungen, beispielsweise alle drei Überprüfungen, positiv, so wird in einigen erfindungsgemäßen Ausführungsformen die Desinfektion fortgesetzt.

In bestimmten erfindungsgemäßen Ausführungsformen umfasst das Verfahren ein Hinzurechnen des vorgegebenen ersten Volumens zur Ultrafiltrationsmenge, insbesondere vor Beginn der Blutbehandlung.

In manchen erfindungsgemäßen Ausführungsformen ist die Blutbehandlungsvorrichtung zur Dialyse, zur Akutdialyse, zur chronischen Dialyse, oder zur chronischen Hämodialyse, insbesondere zur *double needle* Dialyse ausgestaltet.

In bestimmten erfindungsgemäßen Ausführungsformen umfasst das Verfahren die einzelnen Schritte in der hierin angegebenen oder in der alphabetischen Reihenfolge ihrer Bezeichnung.

In einigen erfindungsgemäßen Ausführungsformen wird jeder einzelne Schritte automatisch ausgelöst und durchgeführt.

In bestimmten erfindungsgemäßen Ausführungsformen des Verfahrens folgen manche (beliebige) oder alle der einzelnen Schritte jeweils ohne Zwischenschritt auf den vorangegangen Schritt.

In manchen erfindungsgemäßen Ausführungsformen ist vorgesehen, den jeweils als nächstes anstehenden Schritt nicht zu beginnen, bevor der jeweils vorangegangene Schritt beendet ist. In bestimmten erfindungsgemäßen Ausführungsformen ist vorgesehen, zwischen einigen oder allen der jeweils unmittelbar aufeinander folgenden Schritte des erfindungsgemäßen Verfahrens, hier exemplarisch als früherer und späterer Schritt bezeichnet, nach Abschluss der früheren Schritts eine Mindestzeit (die Mindestzeit kann eine Zeitverzögerung vorbestimmter Dauer sein, in welcher das System nicht reagieren oder voranschreiten kann) abzuwarten, bevor der spätere Schritt ausgeführt werden darf. Ein Beispiel für den früheren Schritt ist insbesondere der Schritt e). Das Verstreichen der Mindestzeit, wo vorgesehen, kann erfindungsgemäß durch jede geeignete Sperrung des Systems sichergestellt werden, etwa durch ein vorübergehendes Stromlosschalten des entsprechenden Systems oder der entsprechenden Einrichtung. Die erfindungsgemäße Steuer- oder Regelvorrichtung kann entsprechend programmiert sein. Auf diese Weise kann es vorteilhaft möglich sein, unerwünschte Druckverhältnisse, welche beispielsweise durch eine bereits bei geschlossener Klemme anlaufende Pumpe entstehen und zur Gefährdung der Patientensicherung durch möglicherweise platzende Leitung, oder beispielsweise zu nachteiligen Wirkungen auf das dem erhöhten Druck ausgesetzte Blut führen können, vorteilhaft zu vermeiden. So kann beispielsweise vorgesehen sein, dass eine (beliebige) Pumpe erst zuverlässig in einen Stillstand der bewegten Pumpenbauteile gebracht werden muss, bevor der sich anschließende Schritt begonnen werden kann. Beispielsweise kann auch vorgesehen sein, eine Klemme erst zu öffnen oder zu schließen, wenn eine mit der Klemme in Fluidkommunikation stehende Pumpe verbindlich und restlos gestoppt wurde. Hierzu kann das Abwarten der Mindestzeit vorteilhaft beitragen. Die Mindestzeit kann einen Wert zwischen 0,5 sec und 20 sec (Sekunden), vorzugsweise zwischen 1 und 10 sec, weiter vorzugsweise zwischen 2 und 5 sec betragen.

Die Mindestzeit kann fest vorgegeben sein, sie kann aber auch variabel und beispielsweise in Abhängigkeit von Sensorergebnissen usw. sein.

In einigen erfindungsgemäßen Ausführungsformen ist der Blutkreislauf oder der Blutschlauchsatz während der Durchführung des erfindungsgemäßen Verfahrens nicht kurzgeschlossen, insbesondere nicht mittels eines Verbinders, welcher beispielsweise den venösen Abschnitt mit dem arteriellen Abschnitt verbindet.

Jede der hierin genannten Einrichtungen der erfindungsgemäßen Vorrichtung kann eigens eingerichtet, konfiguriert und/oder programmiert, angesteuert oder auf andere Weise vorbereitet sein, um den jeweiligen Verfahrensschritt auszuführen, auch wenn dies nicht eigens hierin ausgeführt ist.

Für jeden hierin beschriebenen Verfahrensschritt weisen manche der erfindungsgemäßen Vorrichtungen die zu seiner Ausführung jeweils erforderliche Einrichtung auf.

Die zu der erfindungsgemäßen Vorrichtung getroffenen Feststellungen und Definitionen treffen immer dort, wo dies für den Fachmann zu keinem Widerspruch führt, auch auf das erfindungsgemäße Verfahren zu, und umgekehrt.

Manche oder alle erfindungsgemäßen Ausführungsformen können einen, mehrere oder alle der oben und/oder im Folgenden genannten Vorteile aufweisen.

So kann beispielsweise die vom Dialysepersonal für das Abrüsten, Spülen usw. der verwendeten Blutbehandlungsvorrichtung aufzubringende Zeit vorteilhaft verringert werden. Ein verbesserte Zeitmanagement und ein Potential zum Einsparen von Arbeitszeit des Personals kann hieraus resultieren. Zudem lassen sich Fehler, welche stets mit der manuellen Ausführung von Arbeitsschritten einhergehen können, vermeiden, beispielsweise indem Arbeitsschritte nicht vergessen oder in verkehrte Reihenfolge ausgeführt werden können.

In manchen erfindungsgemäßen Ausführungsformen kann vorteilhaft auf das Kurzschließen bestimmter Teile der Blutbehandlungsvorrichtung, insbesondere eines venösen Abschnitts mit einem arteriellen Abschnitt, beispielsweise des Blutschlauchsatzes und/oder des Blutkreislaufes, verzichtet werden.

Des weiteren lässt sich erfindungsgemäß die Hygiene verbessern, da eine wiederholte Verbindung von ehemals sterilen Komponenten nicht erforderlich ist, etwa beim/durch das Umstecken des arteriellen Abschnitts auf die sog. *safeline.*

Des weiteren erlaubt die vorliegende Erfindung eine verbesserte Mensch-Maschine-Interaktion. Beispielsweise wird die Genauigkeit der Bedienung der erfindungsgemäßen Vorrichtung verbessert, etwa durch das Einhalten von Mindestzeiten, siehe oben.

Schließlich wird die gedankliche Anstrengung des Bedieners bei der Durchführung des erfindungsgemäßen Verfahrens vorteilhaft gesenkt.

Die vorliegende Erfindung wird im Folgenden anhand der beigefügten Zeichnung, in welcher identische Bezugszeichen gleiche oder ähnliche Komponenten bezeichnen, exemplarisch erläutert. In den zum Teil stark vereinfachten Figuren gilt:
- **Fig. 1**: zeigt schematisch vereinfacht eine erfindungsgemäße Vorrichtung zum Durchführen des erfindungsgemäßen Verfahrens; und
- **Fig. 2**: zeigt schematisch in Flussdiagramm die Schritte des erfindungsgemäßen Verfahrens in einer ersten exemplarischen Ausführungsform.

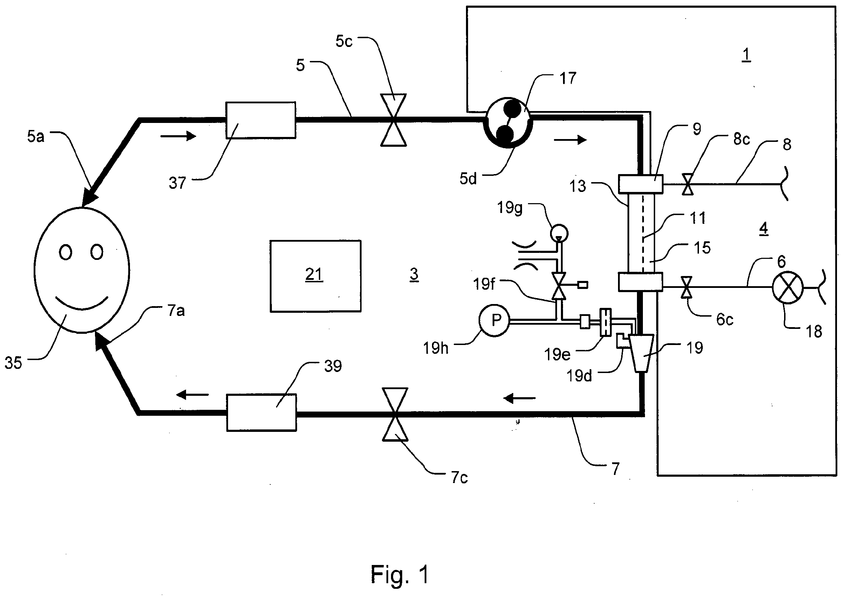

**Fig. 1** zeigt schematisch vereinfacht eine erfindungsgemäße Blutbehandlungsvorrichtung 1, welche verbunden ist mit einem Blutschlauchsatz 3. Der Blutschlauchsatz 3 weist einen arterieller Abschnitt 5 mit einer arteriellen Nadel 5a und einer arteriellen Patientenschlauchklemme 5c auf. Der Blutschlauchsatz 3 weist ferner einen venösen Abschnitt 7 mit einer venösen Nadel 7a und einer venösen Patientenschlauchklemme 7c auf.

Der Blutschlauchsatz 3 weist einen Blutfilter 9 mit einer Membran 11, beispielsweise einer semi-permeablen Membran, auf. Die Membran 11 unterteilt den Blutfilter 9 in eine Blutkammer 13 und eine Dialysierflüssigkeitskammer 15.

Die Blutbehandlungsvorrichtung 1 weist eine Blutpumpe 17 auf. Der Blutschlauchsatz 3 weist eine venöse Tropfkammer 19 auf. Die Blutbehandlungsvorrichtung 1 weist eine Steuer- oder Regelvorrichtung 21 auf oder ist hiermit verbunden. Die Steuer- oder Regelvorrichtung 21 ist mit allen Komponenten der Blutbehandlungsvorrichtung 1 oder des Blutschlauchsatz 3, oder beiden, verbunden, auf welche sie zur Ausführung des erfindungsgemäßen Verfahrens einwirken muss.

Sowohl die arterielle Nadel 5a als auch die venöse Nadel 7a sind mit dem Gefäßsystem eines Patienten 35 verbunden. Der Blutschlauchsatz 3 weist optional einen arteriellen Luftblasendetektor 37 oder einen venösen Blutsensor 39, oder beide, auf.

Die Blutbehandlungsvorrichtung 1 weist ferner eine Fördereinrichtung mit wenigstens einer Dialysierflüssigkeitspumpe 18 zum Fördern von Dialysierflüssigkeit innerhalb eines geschlossenen oder offenen Dialysierflüssigkeitskreislaufs 4 auf. Der Dialysierflüssigkeitskreislauf 4 weist eine Dialysierflüssigkeitszulaufleitung 6, welche mittels einer Dialysierflüssigkeitszulaufleitungsklemme 6c der Blutbehandlungsvorrichtung 1 unterbrochen werden kann, und eine Dialysatablaufleitung 8, welche mittels einer Dialysatablaufleitungsklemme 8c der Blutbehandlungsvorrichtung 1 unterbrochen werden kann, auf. Die Steuer- oder Regelvorrichtung 21 ist mit allen Komponenten der Fördereinrichtung oder des Dialysierflüssigkeitskreislaufs 4, oder beiden, verbunden, auf welche sie zur Ausführung des erfindungsgemäßen Verfahrens einwirken muss.

Der arterieller Abschnitt 5 des Blutschlauchsatzes 3 weist ein Blutschlauchsegment 5d auf.

Die venöse Tropfkammer 19 weist ein Belüftungsventil 19d auf.

Die venöse Tropfkammer 19 ist ferner mit einer Leitung oder Verbindungsleitung verbunden, welche über einen optional vorgesehenen Sterilfilter 19e über ein optional vorhandenes Ventil 19f mit einem Kompressor 19g verbunden ist. Dieselbe Leitung ist in der hier gezeigten beispielhaften Ausführungsform ferner mit einem venösen Drucksensor 19h verbunden.

Die Elemente 19e bis 19h sind aus Gründen der Darstellung innerhalb des Blutschlauchsatzes 3 gezeigt. Sie können jedoch einzelnen oder gemeinsam auch Teil der Blutbehandlungsvorrichtung 1 sein. Dies gilt insbesondere für den Kompressor 19g, das Ventil 19f und den Drucksensor 19h.

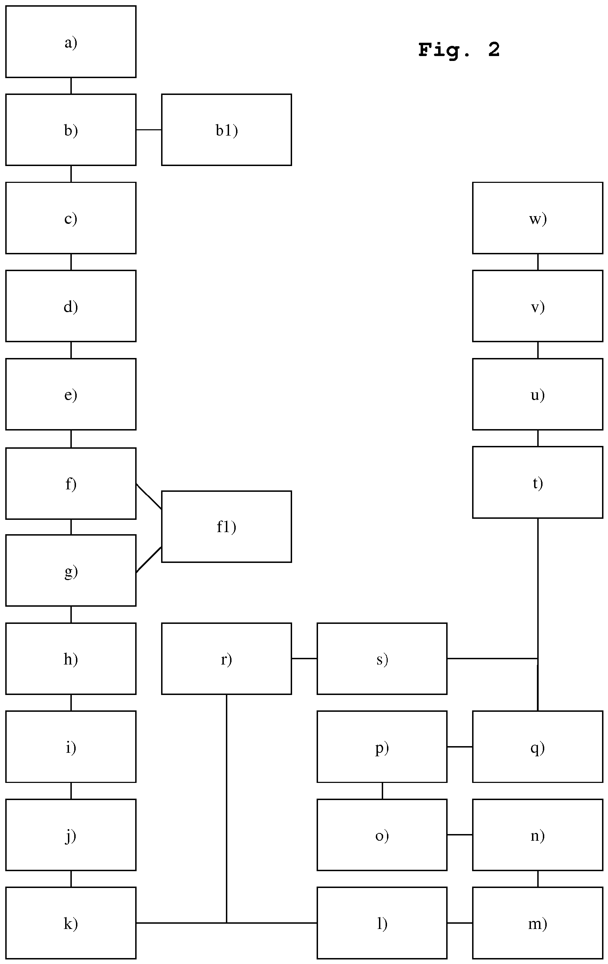

**Fig. 2** zeigt schematisch vereinfacht als Flussdiagramm den Ablauf des erfindungsgemäßen Verfahrens in einer ersten exemplarischen Ausführungsform.

Das Verfahren beginnt in dieser Ausführungsform in Schritt a) mit dem Schließen der arteriellen Patientenschlauchklemme 5c und der venösen Patientenschlauchklemme 7c.

Im Schritt b) wird der Fluss der mittels der Dialysierflüssigkeitspumpe 18 geförderten Dialysierflüssigkeit automatisch auf einen voreingestellten Wert reduziert oder eingestellt. Der voreingestellte Wert kann jenem für den *rinse back*-Vorgang bekannten entsprechen (z. B. 100 ml/min). Schritt b) kann insbesondere unmittelbar auf Schritt a) folgen.

Der optionale Schritt b1) sieht vor, dass in Schritt b) der Dialyierflüssigkeitsfluss konstant oder nicht konstant ist. Letzteres kann beispielsweise mittels einer pulsierenden Veränderung erfolgen, mit dem Ziel, angelagerte Zell- und Protein-Bestandteile im Faservolumen des Blutfilters 9 zu lösen.

Im Schritt c) wird die venöse Patientenschlauchklemme 7c geöffnet, welche in Schritt a) zum Erzielen eines sog. sicheren Zustands der Blutbehandlungsvorrichtung im Anschluss an die Blutbehandlung geschlossen wurde.

Im Schritt d) wird der Ausgang des Blutfilters 9 für Dialysat geschlossen. Dies kann beispielsweise durch Schließen einer Dialysatablaufleitungsklemme 8c einer Dialysatablaufleitung 8 erfolgen. Somit tritt Dialysierflüssigkeit aus dem Dialysierflüssigkeitskreislauf 4 über die Membran 11 ins Lumen des Blutfilters 9 und schließlich über den venösen Abschnitt 7 in das Gefäßsystem des Patienten 35 ein.

Schritt e) sieht ein Stoppen, Unterbrechen oder Unterbinden des mittels der Dialysierflüssigkeitspumpe 18 erzielten Dialysierflüssigkeitsflusses vor. Dies kann durch Beeinflussen oder Anhalten der Dialysierflüssigkeitspumpe 18 erfolgen. Alternativ oder ergänzend kann dies auch durch eine Veränderung oder Unterbrechung des Stromweges der Dialysierflüssigkeit geschehen.

Das Stoppen, Unterbrechen oder Unterbinden erfolgt in bestimmten erfindungsgemäßen Ausführungsformen insbesondere dann, wenn (sobald) ein erstes Volumen abzüglich eines zweiten Volumens über die Membran 11 übergetreten ist. Das erste Volumen kann einem voreingestellten Wert entsprechen, welcher beispielsweise vom Dialysepersonal an der Behandlungsvorrichtung festgelegt wurde. Sein Wert kann wie oben definiert sein. Seine Größe kann automatisch von der Behandlungsmaschine ermittelt und/oder aus Listen entnommen werden. Das zweite Volumen, auch als ein Delta bezeichnet, entspricht dem Volumen des gesamten arteriellen Abschnitts 5 des extrakorporalen Blutkreislaufs 3 bis zum Eingang des Blutfilters (9).

Vor, mit oder nach Schritt e) wird in einem Schritt f) die venöse Patientenschlauchklemme 7c geschlossen.

Im wiederum optionalen Schritt f1) wird - im Zuge einer alternativen Möglichkeit - ein in die Blutpumpe 17 eingelegtes Blutschlauchsegment 5d des Blutschlauchsatzes 3 durch eine geeignete Einrichtung, beispielsweise den sog. ALPHA-clip, ausgeworfen oder auf andere Weise automatisch von der Blutpumpe 17 gelöst.

Im anschließenden Schritt g) wird Dialysierflüssigkeit mittels der Dialysierflüssigkeitspumpe 1 gefördert, bei zuvor, gleichzeitig oder nachträglich geöffneter arterieller Patientenschlauchklemme 5c (Schritt h) und weiterhin geschlossenem Dialysatablauf, etwa bei geschlossener Dialysatablaufleitung 8.

Im optionalen Schritt i) wird die Fördergeschwindigkeit der Dialysierflüssigkeitspumpe 18 verringert. Es lösen sich hierdurch vorteilhaft möglichst wenig Gerinnsel. Die verringerte Fördergeschwindigkeit kann wiederum beispielsweise 50% der Behandlungs-Fördergeschwindigkeit betragen, siehe oben.

Im Schritt j) wird die arterielle Patientenschlauchklemme 5c geschlossen.

Im Schritt k) wird die Dialysierflüssigkeitspumpe 18 gestoppt oder - alternativ oder ergänzend - eine andere Maßnahme zum Unterbinden des Dialysierflüssigkeitsstroms getroffen, sobald das zweite vorbestimmte Volumen mittels der Dialysierflüssigkeitspumpe 18 gefördert wurde. Das zweite, vorbestimmte Volumen kann das arterielle *rinse back* Volumen sein. Alternativ wird gefördert bis Luft oder Gerinnsel mittels eines jeweils entsprechenden Sensors erkannt wurde.

Das Verfahren umfasst weiter optional die folgenden Schritte oder alternativ die Schritte l) bis q) oder die Schritte r) bis s):
Im Schritt l) wird beispielsweise eine nicht dargestellte, stromab des Blutfilters 9 gelegene Ultrafiltrationspumpe oder eine ebenfalls nicht dargestellte Dialysierflüssigkeitspumpe (oder, korrekter: Dialysatpumpe) angefahren. Die Dialysierflüssigkeitszulaufsleitung 6 wird geschlossen gehalten (Schritt m)). Die Dialysierflüssigkeitsleitungsklemme 8c wird geöffnet. Die arterielle und die venöse Patientenschlauchklemme 5c, 7c werden geschlossen gehalten (Schritt o)).

In manchen erfindungsgemäßen Ausführungsformen wird für den Schritt l) die Ultrafiltrationspumpe (in den Figuren nicht gezeigt) zum Leeren gemäß Schritt l) in Betrieb gesetzt. Wiederum alternativ wird in bestimmten erfindungsgemäßen Ausführungsformen für Schritt l) der Kompressor 19g (siehe Fig. 1) in Betrieb gesetzt. Schließlich können auch ein kombiniertes Verwenden von (jeweils beliebigen) zwei oder drei der vorgenannten Pumpen zum Leeren des Lumens in Schritt l) verwendet werden.

Im Schritt p) wird ein Belüftungsventil 19d geöffnet. Rein exemplarisch kann das Belüftungsventil in der venösen Tropfkammer 19 oder in einer Verbindungsleitung zur venösen Tropfkammer 19, welche einen venösen Druckwandler und/oder einen venösen Drucksensor 19h aufweist, vorgesehen sein.

Mittels der Dialysierflüssigkeitspumpe 18 wird im Schritt q) Dialysierflüssigkeit über die Membran 11 aus dem Blutschlauchsatz 3 gesaugt.

Im optionalen Schritt t) wird der Behälter mit Bikarbonat-Trockenkonzentrat (nicht dargestellt) entleert.

In den alternativen oder ergänzenden Schritten r) bis s) wird die Dialysatablaufleitung 8 geöffnet (Schritt r)).

Im Schritt s) wird Gas, beispielsweise Luft, in die Verbindungsleitung mit dem venösen Drucksensor 19h oder an anderer Stelle, eingebracht, beispielsweise mittels des Kompressors 19h. Das Gas verdrängt das Dialysat über die Membran 11 hindurch in die Dialysierflüssigkeitskammer 15. Von dort gelangt es über die Dialysatablaufleitung und ein geöffnete Ventil in den Abfluss.

Mit Schritt t) wird der Behälter mit Bikarbonat-Trockenkonzentrat entleert.

Das Verfahren umfasst dann wieder ein Stoppen der Dialysierflüssigkeitspumpe 18 (Schritt u)), das Schließen von arterieller Patientenschlauchklemme 5c, venöser Patientenschlauchklemme 7c, ggf. auch der Substituatklemme (Infusatklemme), soweit noch nicht erfolgt (Schritt v) und das Ausgeben eines Alarms oder Hinweises zum Informieren des Dialysepersonals über das Verfahrensende (Schritt w). Der Schritt w) ist in bestimmten erfindungsgemäßen Ausführungsformen der das Verfahrens abschließende Schritt. Er ist in einigen erfindungsgemäßen Ausführungsformen bereits im Abschluss an einen der Schritte k) bis u) vorgesehen.

Es wird darauf hingewiesen, dass die Abfolge der Schritte, wie sie mit Bezug auf Fig. 2 oder auch in den angehängten Ansprüchen dargelegt ist, nur beispielhaft ist. Tatsächlich können Schritte, welche zwar nacheinander beschreiben sind, erfindungsgemäß auch in anderer oder umgekehrter Reihenfolge ablaufen, soweit aus Sicht des Fachmann nichts hiergegen spricht. Dies gilt insbesondere für die Schritte c), d) und e). Die vorliegende Erfindung umfasst auch andere Reihenfolgen, ferner auch ihr paralleles Ablaufen. Vorstehende Erläuterungen gelten auch für die Schritte i) und g). Auch sie können parallel oder in umgekehrter Reihenfolge durchgeführt werden.

**Bezugszeichenliste**

| **Bezugszeichen** | **Beschreibung** |
|---|---|
| 1 | Blutbehandlungsvorrichtung |
| 3 | Blutschlauchsatz |
| 4 | Dialysierflüssigkeitskreislauf |
| 5 | arterieller Abschnitt |
| 5a | arterielle Nadel |
| 5c | arterielle Patientenschlauchklemme |
| 5d | Blutschlauchsegment |
| 6 | Dialysierflüssigkeitszulaufleitung |
| 6c | Dialysierflüssigkeitszulaufleitungsklemme |
| 7 | venöser Abschnitt |
| 7a | venöse Nadel |
| 7c | venöse Patientenschlauchklemme |
| 8 | Dialysatablaufleitung |
| 8c | Dialysatablaufleitungsklemme |
| 9 | Blutfilter |
| 11 | Membran |
| 13 | Blutkammer |
| 15 | Dialysierflüssigkeitskammer |
| 17 | Blutpumpe |
| 18 | Dialysierflüssigkeitspumpe |
| 19 | venöse Tropfkammer |
| 19d | Belüftungsventil |
| 19e | Sterilfilter |
| 19f | Ventil |
| 19g | Kompressor |
| 19h | Drucksensor |
| 21 | Steuer- oder Regelvorrichtung |
| 35 | Patient |
| 37 | Luftblasendetektor |
| 39 | venöser Blutsensor |

## Patentansprüche

1. Steuer- oder Regelvorrichtung (21), konfiguriert, um im Zusammenwirken mit einer Blutbehandlungsvorrichtung (1), welche eine Blutpumpe (17) zum Fördern von Blut innerhalb eines extrakorporalen Blutschlauchsatzes (3) und eine Dialysierflüssigkeitspumpe (18) zum Fördern von Dialysierflüssigkeit innerhalb eines Dialysierflüssigkeitskreislaufs (4), welcher eine Dialysierflüssigkeitszulaufleitung (6), welche mittels einer Dialysierflüssigkeitszulaufleitungsklemme (6c) der Blutbehandlungsvorrichtung (1) unterbrochen werden kann, und eine Dialysatablaufleitung (8), welche mittels einer Dialysatablaufleitungsklemme (8c) der Blutbehandlungsvorrichtung (1) unterbrochen werden kann, umfasst, aufweist,
wobei der Blutschlauchsatz (3) einen arteriellen Abschnitt (5) aufweist, welcher eine arterielle Patientenschlauchklemme (5c) aufweist oder hiermit verbunden ist;
wobei der Blutschlauchsatz (3) einen venösen Abschnitt (7) aufweist, welcher eine venöse Patientenschlauchklemme (7c) aufweist oder hiermit verbunden ist,
wobei der Blutschlauchsatz (3) einen zwischen dem arteriellen Abschnitt (5) und dem venösen Abschnitt (7) angeordneten Blutfilter (9) mit einer Membran (11), welche eine Blutkammer (13) von einer Dialysierflüssigkeitskammer (15) trennt, aufweist oder hiermit verbunden ist,
ein Verfahren zum Steuern der Blutbehandlungsvorrichtung (1) auszuführen oder zu veranlassen,
wobei das Verfahren die automatisch durchgeführten Schritte umfasst:
a) Schließen der arteriellen Patientenschlauchklemme (5c) und der venösen Patientenschlauchklemme (7c);
b) Fördern von Dialysierflüssigkeit mittels der Dialysierflüssigkeitspumpe (18);
c) Öffnen der venösen Patientenschlauchklemme (7c);
d) Schließen der Dialysatablaufleitung (8);
e) Unterbinden oder Beenden des Dialysierflüssigkeitsflusses, sobald ein vorgegebenes erstes Volumen abzüglich eines zweiten Volumens über die Membran (11) übergetreten ist, wobei das zweite Volumen vorzugsweise dem Volumen des arteriellen Abschnitts (5) des extrakorporalen Blutkreislaufs (3) bis zum Eingang des Blutfilters (9) entspricht;
f) Schließen der venösen Patientenschlauchklemme (7c);
g) Fördern von Dialysierflüssigkeit mittels der Dialysierflüssigkeitspumpe (18);
h) Öffnen der arteriellen Patientenschlauchklemme (5c) bei weiterhin geschlossener Dialysatablaufleitung (8);
j) Schließen der arteriellen Patientenschlauchklemme (5c);
k) Stoppen der Dialysierflüssigkeitspumpe (18) sobald das zweite vorbestimmte Volumen gefördert wurde oder Luft oder Gerinnsel mittels eines Sensors erkannt wurde;
wobei das Verfahren weiter die folgenden Schritte oder alternativ die Schritte l) bis q) oder die Schritte r) bis s) umfasst:
l) Anfahren der Dialysierflüssigkeitspumpe (18) oder einer stromab des Blutfilters (9) liegenden Dialysierflüssigkeitspumpe oder einer stromab des Blutfilters (9) liegenden Ultrafiltrationspumpe;
m) Schließen oder Geschlossenhalten der Dialysierflüssigkeitszulaufsleitung (6);
n) Öffnen der Dialysatsablaufleitungsklemme (8c);
p) Öffnen eines Belüftungsventils;
q) Saugen von Dialysierflüssigkeit mittels der Dialysierflüssigkeitspumpe (18) über die Membran (11) aus dem Blutschlauchsatz (3);
r) Öffnen der Dialysatablaufleitung (8);
s) Einbringen von Gas in eine Verbindungsleitung zum venösen Drucksensor (19h), welches das Dialysat über die Membran (11) auf die Dialysatausgangsseite verdrängt;
u) Stoppen der Dialysierflüssigkeitspumpe (18); und
w) Ausgeben eines Alarms oder Hinweises zum Informieren des Dialysepersonals über das Verfahrensende.

2. Steuer- oder Regelvorrichtung (21) nach Anspruch 1, konfiguriert zum Ausführen oder Veranlassen des den Schritten des Verfahrens aus Anspruch 1 vorgehenden automatischen Schritts:
a0) Stoppen der Blutpumpe (17) zum Beenden einer dem Verfahren gemäß Anspruch 1 vorangegangenen Blutbehandlung, wenn eine der folgenden Bedingungen erfüllt ist:
- Erreichen einer vorgegebenen Zeitpunkts;
- Erreichen einer vorgegebenen Behandlungsdauer;
- Erreichen eines vorgegebenen Dialysedosiswerts; oder
- Erreichen einer vorbestimmten Ultrafiltrationsmenge.

3. Steuer- oder Regelvorrichtung (21) nach Anspruch 1 oder 2, wobei das darin genannte Verfahren, zu dessen Ausführen oder Veranlassen die Steuer- oder Regelvorrichtung (21) konfiguriert ist, den weiteren automatischen Schritt umfasst:
f1) Auswerfen des Blutschlauchsegments (5d) des Blutschlauchsatzes (3).

4. Steuer- oder Regelvorrichtung (21) nach einem der Ansprüche 1 bis 3, wobei in Schritt b) des darin genannten Verfahrens, zu dessen Ausführen oder Veranlassen die Steuer- oder Regelvorrichtung (21) konfiguriert ist, die Dialyierflüssigkeitsrate automatisch verändert wird.

5. Steuer- oder Regelvorrichtung (21) nach einem der Ansprüche 1 bis 4, wobei das darin genannte Verfahren, zu dessen Ausführen oder Veranlassen die Steuer- oder Regelvorrichtung (21) konfiguriert ist, einen oder mehrere der automatischen Schritte umfasst:
- Trennen der Dialysatkonnektoren und/oder eines online Konnektors oder Unterbinden einer Fluidkommunikation zwischen diesen und der Hydraulik mittels Ventile;
- Trennen des Behälters mit Bikarbonat-Trockenkonzentrat von der Hydraulik oder Unterbinden einer Fluidkommunikation zwischen Behälter und Hydraulik.

6. Steuer- oder Regelvorrichtung (21) nach Anspruch 5, wobei die Desinfektion nach Erkennen, dass die Dialysatschläuche an der Maschine konnektiert wurden, der online-Konnektor in Spülstellung ist und der Behälter mit Bikarbonat-Trockenkonzentrat entfernt wurde, automatisch fortgesetzt und beendet wird.

7. Steuer- oder Regelvorrichtung (21) nach einem der Ansprüche 1 bis 6, wobei das Verfahren, zu dessen Ausführen oder Veranlassen die Steuer- oder Regelvorrichtung (21) konfiguriert ist, den automatischen Schritt umfasst:
a00) Hinzurechnen des vorgegebenen ersten Volumens zur Ultrafiltrationsmenge.

8. Blutbehandlungsvorrichtung (1), welche eine Steuer- oder Regelvorrichtung (21) nach einem der vorangegangenen Ansprüche aufweist.

9. Blutbehandlungsvorrichtung (1) nach Anspruch 8, welche zur Dialyse, zur Akutdialyse, zur chronischen Dialyse, oder zur chronischen Hämodialyse, insbesondere zur *double needle* Dialyse, ausgestaltet ist.

## Claims

1. A control or regulating apparatus (21) configured,
to execute or initiate a process for controlling the blood treatment apparatus (1),
in cooperation with a blood treatment apparatus (1), which comprises a blood pump (17) for conveying blood within an extracorporeal blood tubing set (3) as well as a dialysis liquid pump (18) for conveying dialysis liquid within a dialysis liquid circuit (4), which comprises a dialysis liquid feed line (6), which can be interrupted by means of a dialysis liquid feed line clamp (6c) of the blood treatment apparatus (1), and a dialysate drain line (8), which can be interrupted by means of a dialysate drain line clamp (8c) of the blood treatment apparatus (1),
wherein the blood tubing set (3) comprises an arterial portion (5) comprising an arterial patient tubing clamp (5c) or being connected thereto;
wherein the blood tubing set (3) comprises a venous portion (7) comprising a venous patient tubing clamp (7c) or being connected thereto;
wherein the blood tubing set (3) comprises or is connected to a blood filter (9) having a membrane (11) which separates a blood chamber (13) from a dialysis fluid chamber (15), and which is arranged between the arterial portion (5) and the venous portion (7),
wherein the process comprises the automatically performed steps of:
a) closing the arterial patient tubing clamp (5c) and the venous patient tubing clamp (7c);
b) conveying dialysis liquid by means of the dialysis liquid pump (18);
c) opening the venous patient tubing clamp (7c);
d) closing the dialysate drain line (8);
e) preventing or ending the dialysis liquid flow, as soon as a predefined first volume, after deduction of a second volume, has passed over the membrane (11), the second volume preferably corresponding to the volume of the arterial portion (5) of the extracorporeal blood circuit (3) up to the inlet of the blood filter (9);
f) closing the venous patient tubing clamp (7c);
g) conveying dialysis liquid by means of the dialysis liquid pump (18);
h) opening the arterial patient tubing clamp (5c) while the dialysate drain line (8) remains closed;
j) closing the arterial patient tubing clamp (5c);
k) stopping the dialysis liquid pump (18) as soon as the second predetermined volume has been conveyed or air or clot has been detected by a sensor;
wherein the process further comprises the following steps or alternatively steps 1) to q) or steps r) to s):
1) starting up the dialysis liquid pump (18) or a dialysis liquid pump located downstream of the blood filter (9) or an ultrafiltration pump located downstream of the blood filter (9);
m) closing or keeping closed the dialysis liquid feed line (6);
n) opening the dialysate drain line clamp (8c);
p) opening a ventilation valve;
q) sucking dialysis liquid out of the blood tubing set (3) via the membrane (11) by means of the dialysis liquid pump (18);
r) opening the dialysate drain line (8);
s) introducing gas into a connecting line to the venous pressure sensor (19h), which displaces the dialysate through the membrane (11) to the dialysate outlet side;
u) stopping the dialysis liquid pump (18); and
w) issuing an alarm or an indication to inform dialysis personnel about the end of the process.

2. The control or regulating apparatus (21) according to claim 1, configured to perform or initiate the following automatic step preceding the steps of claim 1 of the process:
a0) stopping the blood pump (17) to end a blood treatment preceding the process according to claim 1, when one of the following conditions is satisfied:
- reaching a predefined point of time;
- reaching a predefined treatment duration;
- reaching a predefined dialysis dose value; or
- reaching a predetermined amount of ultrafiltration.

3. The control or regulating apparatus (21) according to claim 1 or 2, wherein the process mentioned therein for the execution or initiation of which the control or regulating apparatus (21) is configured, comprises the further automatic step of:
fl) ejecting the blood tubing segments (5d) of the blood tubing set (3).

4. The control or regulating apparatus (21) according to anyone of claims 1 to 3, wherein in step b) of the process mentioned therein for the execution or initiation of which the control or regulating apparatus (21) is configured, the dialysis fluid rate is changed automatically.

5. The control or regulating apparatus (21) according to anyone of claims 1 to 4, wherein the process mentioned therein for the execution or initiation of which the control or regulating device (21) is configured, comprises one or more of the automatic steps of:
- disconnecting the dialysate connectors and/or an online connector or preventing a fluid communication between these and the hydraulics by means of valves;
- disconnecting the container with bicarbonate dry concentrate from the hydraulics or preventing a fluid communication between the container and the hydraulics.

6. The control or regulating apparatus (21) according to claim 5, wherein the disinfection continues and ends automatically after detecting that the dialysate tubings have been connected to the machine, the online connector is in rinsing position and the container with bicarbonate dry concentrate has been removed.

7. The control or regulating apparatus (21) according to anyone of claims 1 to 6, wherein the process for the execution or initiation of which the control or regulating device (21) is configured, comprises the automatic step of:
a00) adding the predefined first volume to the ultrafiltration amount.

8. A blood treatment apparatus (1), which comprises a control or regulating apparatus (21) according to anyone of the preceding claims.

9. The blood treatment apparatus (1) according to claim 8, designed for dialysis, for acute dialysis, for chronic dialysis, or for chronic haemodialysis, in particular for *double needle* dialysis.

## Revendications

1. Un appareil de commande ou de régulation (21), configuré
pour exécuter ou déclencher un procédé de commande de l'appareil de traitement du sang (1),
en coopération avec un appareil de traitement du sang (1), qui comprend une pompe à sang (17), pour acheminer le sang dans un set de tuyaux sanguins extracorporels (3) ainsi qu'une pompe à liquide de dialyse (18) pour acheminer le liquide de dialyse dans un circuit de liquide de dialyse (4) comprenant un conduit d'alimentation en liquide de dialyse (6) qui peut être interrompu au moyen d'une pince de serrage de conduit d'alimentation en liquide de dialyse (6c) de l'appareil de traitement du sang (1)
ainsi qu'un conduit d'évacuation de dialysat (8) qui peut être interrompu au moyen d'une pince de serrage de conduit d'évacuation de dialysat (8c) de l'appareil de traitement du sang (1),
où le set de tuyaux sanguins (3) comprend une section artérielle (5) comprenant une pince de serrage de tuyaux artériels du patient (5c), ou est raccordé à celle-ci;
où le set de tuyaux sanguins (3) comprend une section veineuse (7) comprenant une pince de serrage de tuyaux veineux du patient (7c), ou est raccordé à celle-ci;
où le set de tuyaux sanguins (3) comprend ou est raccordé à un filtre sanguin (9) agencé entre la section artérielle (5) et la section veineuse (7) et doté d'une membrane (11) séparant une chambre à sang (13) d'une chambre à liquide de dialyse (15),
où le procédé comprend les étapes exécutées automatiquement consistant à:
a) fermer la pince de serrage des tuyaux artériels du patient (5c) ainsi que la pince de serrage des tuyaux veineux du patient (7c);
b) acheminer le liquide de dialyse au moyen de la pompe à liquide de dialyse (18);
c) ouvrir la pince de serrage de tuyaux veineux du patient (7c);
d) fermer le conduit d'évacuation de dialysat (8);
e) empêcher ou arrêter le flux du liquide de dialyse dès lors qu'un premier volume prédéfini, après déduction d'un second volume, a traversé la membrane (11), le second volume correspondant de préférence au volume de la section artérielle (5) du circuit sanguin extracorporel (3) jusqu'à l'entrée du filtre à sang (9);
f) fermer la pince de serrage des tuyaux veineux du patient (7c);
g) acheminer le liquide de dialyse au moyen de la pompe à liquide de dialyse (18);
h) ouvrir la pince de serrage des tuyaux artériels du patient (5c) alors que le conduit d'évacuation de dialysat (8) reste fermé;
j) fermer la pince de serrage des tuyaux artériels du patient (5c);
k) arrêter la pompe à liquide de dialyse (18) dès lors que le second volume prédéterminé a été acheminé, ou que de l'air ou un caillot a été détecté par un capteur;
où le procédé comprend en outre les étapes suivantes, ou alternativement les étapes l) à q) ou les étapes r) à s):
l) mettre en marche la pompe à liquide de dialyse (18) ou une pompe à liquide de dialyse située en aval du filtre à sang (9) ou une pompe d'ultrafiltration située en aval du filtre à sang (9);
m) fermer ou maintenir fermé le conduit d'alimentation en liquide de dialyse (6);
n) ouvrir la pince de serrage du conduit d'évacuation de dialysat (8c);
p) ouvrir une vanne de ventilation;
q) aspirer le liquide de dialyse du set de tuyaux sanguins (3) au travers de la membrane (11) au moyen de la pompe à liquide de dialyse (18);
r) ouvrir le conduit d'évacuation de dialysat (8);
s) introduire du gaz dans un conduit de raccordement au capteur de pression veineuse (19h), lequel déplace le dialysat au travers de la membrane (11) vers le côté de sortie du dialysat;
u) arrêter la pompe à liquide de dialyse (18); et
w) émettre une alarme ou une indication pour informer le personnel de dialyse de la fin du procédé.

2. L'appareil de commande ou de régulation (21) selon la première revendication, configuré pour effectuer ou déclencher l'étape automatique précédant les étapes de la première revendication du procédé consistant à:
a0) arrêter la pompe à sang (17) pour mettre fin à un traitement du sang précédant le procédé selon la première revendication, lorsque l'une des conditions suivantes est remplie:
- atteindre un instant prédéfini;
- atteindre une durée de traitement prédéfinie;
- atteindre une valeur de dose de dialyse prédéfinie; ou
- atteindre une quantité d'ultrafiltration prédéterminée.

3. L'appareil de commande ou de régulation (21) selon la revendication 1 ou 2, où le procédé qui y est mentionné pour l'exécution ou le déclenchement duquel l'appareil de commande ou de régulation (21) est configuré, comprend l'étape automatique supplémentaire consistant à:
fl) éjecter le segment des tuyaux sanguins (5d) du set de tuyaux sanguins (3).

4. L'appareil de commande ou de régulation (21) selon l'une quelconque des revendications 1 à 3, où à l'étape b) du procédé qui y est mentionné pour l'exécution ou le déclenchement duquel l'appareil de commande ou de régulation (21) est configuré, le débit de liquide de dialyse est modifié automatiquement.

5. L'appareil de commande ou de régulation (21) selon l'une quelconque des revendications 1 à 4, où le procédé qui y est mentionné pour l'exécution ou le déclenchement duquel l'appareil de commande ou de régulation (21) est configuré, comprend une ou plusieurs étapes automatiques consistant à:
- déconnecter les connecteurs de dialysat et/ou un connecteur online ou empêcher une communication fluidique entre ceux-ci et le système hydraulique au moyen de vannes;
- séparer le réservoir contenant du concentré sec de bicarbonate du système hydraulique ou empêcher la communication fluidique entre le réservoir et le système hydraulique.

6. L'appareil de commande ou de régulation (21) selon la revendication 5, où la désinfection se poursuit et se termine automatiquement après constat que les tuyaux de dialysat ont été connectés à la machine, que le connecteur online est en position de rinçage et que le réservoir contenant du concentré sec de bicarbonate a été enlevé.

7. L'appareil de commande et de régulation (21) selon l'une quelconque des revendications 1 à 6, où le procédé pour l'exécution ou le déclenchement duquel l'appareil de commande ou de régulation (21) est configuré, comprend l'étape automatique consistant à:
a00) ajouter le premier volume prédéfini à la quantité d'ultrafiltration.

8. Un appareil de traitement du sang (1) qui comprend un appareil de commande ou de régulation (21) selon l'une quelconque des revendications précédentes.

9. L'appareil de traitement du sang (1) selon la revendication 8, conçu pour la dialyse, pour la dialyse aigue, pour la dialyse chronique, ou pour l'hémodialyse chronique, notamment pour la dialyse *à double aiguille.*
